# EUROPEAN PATENT APPLICATION

(11) **EP 4 570 223 A1**
(43) Date of publication of application: **18.06.2025**
(21) Application number: 23865322.4
(22) Date of filing: 31.08.2023
(51) Int. Cl.: A61F 13/534, A61F 13/535, A61F 13/539

(54) **ABSORBENT ARTICLE**

(30) Priority: 12.09.2022 JP 2022144928
(71) Applicant: Zuiko Corporation, Ibaraki-shi Osaka 5670082 (JP)
(72) Inventor: UMEBAYASHI Toyoshi, Ibaraki-shi, Osaka 567-0082 (JP); TANAKA Yasutaka, Ibaraki-shi, Osaka 567-0082 (JP)
(74) Representative: Hasegawa, Kan
(86) International application number: PCT/JP2023/031971
(87) International publication number: WO 2024/057967

(57) **Abstract**

Provided is an absorbent article capable of suppressing leakage of liquid that could not be absorbed from edges in the longitudinal direction.

An absorbent article (10) comprises: a first absorbent layer (20) formed with a crushed fibrous material and extending in the longitudinal direction; and a second absorbent layer (30) formed with a sheet-like material retaining an absorbent resin material and falling into the region of the first absorbent layer (20) in the planar view. The first absorbent layer (20) includes, in the planar view, an overlapping part (21k) that overlaps with the region of the second absorbent layer (30), and protruding parts (21p, 21q) that protrude in the longitudinal direction from the region of the second absorbent layer (30). The absorbent article (10) is used so that the first absorbent layer (20) is positioned on the side of a user and the second absorbent layer (30) is positioned on the opposite side of the user relative to the first absorbent layer (20).

## Description

### TECHNICAL FIELD

The present invention relates to an absorbent article, and more specifically, relates to an absorbent article used for paper diapers and the like.

### BACKGROUND ART

An absorbent article includes an absorbent layer for absorbing and retaining liquid. The absorbent layer is formed with a crushed fibrous material or formed with a sheet-like material retaining an absorbent rein material.

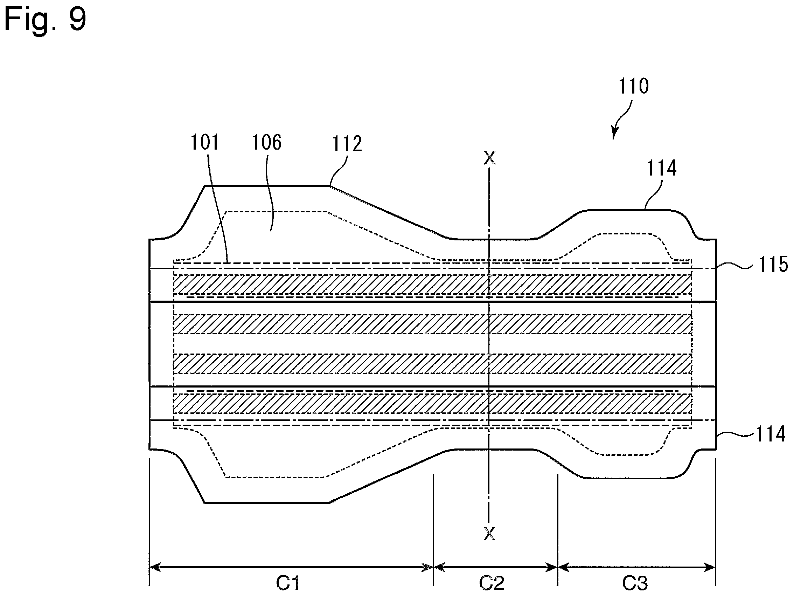

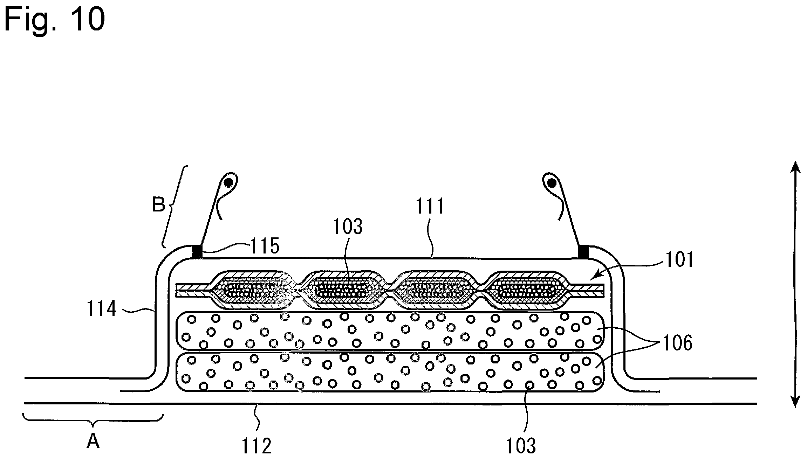

For example, an absorbent article 110 shown in Figs. 9 and 10 includes both of these absorbent layers. Fig. 9 is a plan view of the absorbent article 110. Fig. 10 is a cross-sectional view taken along the line X-X of Fig. 9. The arrow on the right side in Fig. 10 indicates that the upper side is the user's skin surface side.

As shown in Figs. 9 and 10, to form the absorbent article 110, first, an absorbent mat provided with a sheet-like liquid-absorbing layer 101 and fiber assembly layers 106 is sandwiched between a liquid-impermeable rear surface sheet 112 and a liquid-permeable front surface sheet 111 in such a manner that the sheet-like liquid-absorbing layer 101 is on the side of the front surface sheet 111, and then, a liquid-impermeable side sheet 114 is provided so as to cover from the bottom to the side of the absorbent mat. Here, the side sheet 114 is bonded to the front surface sheet 111 and the rear surface sheet 112 in a fixing area A provided in an outward extending part in the bottom part of the absorbent mat, is bonded to the front surface sheet 111 at the edge (base end bonded part 115) in the width direction of the absorbent mat in the upper part (user's side) of the absorbent mat, and has a rising area B where the side sheet 114 rises from the base end bonded part 115.

The fiber assembly layers 106 are formed with fibrillated pulp fibers, thermally fusible fibers and absorbent resin powder 103 dispersed in these fibers, and are formed by wrapping an integration of these with thin paper (for example, tissue paper).

The sheet-like liquid-absorbing layer 101 has a structure in which the absorbent resin powder 103 is held between two sheets of nonwoven fabric.

The wearable article 110 is used so that the sheet-like liquid-absorbing layer 101 is positioned on the user's side and the fiber assembly layers 106 are disposed on the opposite side of the user relative to the sheet-like liquid-absorbing layer 101. The wearable article 110 is substantially gourd-shaped as a whole. That is, to ensure that it adheres closely to the user's groin, an intermediate part C2 in the length direction is narrower than a rear part C1 in the length direction that comes in contact with the user's buttocks, and a front part C3 in the length direction has a spread in the width direction to the extent that does not cause discomfort to the user.

Once the sheet-like liquid-absorbing layer 101 absorbs a body fluid, its thickness increases because of the swelling of the absorbent resin powder 103, so that the distance between the fiber assembly layers 106 as the last water-retaining layers and the user's skin increases (for example, see Patent Literature 1).

### CITATION LIST

### PATENT LITERATURE

[Patent Literature 1] WO 2004/080361 A1

### SUMMARY OF THE INVENTION

### PROBLEM TO BE SOLVED BY THE INVENTION

With the absorbent article shown in Figs. 9 and 10, liquid that cannot be absorbed completely by the absorbent mat can leak from the ends, in the length direction, of the fiber assembly layers 106 and the sheet-like liquid-absorbing layer 101 of the absorbent mat.

In view of such circumstances, a problem to be solved by the present invention is to provide an absorbent article capable of inhibiting leakage of liquid that cannot be absorbed completely, from the ends in the longitudinal direction.

### MEANS FOR SOLVING THE PROBLEM

To solve the above-mentioned problem, the present invention provides an absorbent article structured as follows:

The absorbent article is provided with: (a) a first absorbent layer formed with a crushed fibrous material and extending in a longitudinal direction; and (b) a second absorbent layer formed with a sheet-like material retaining an absorbent resin material and falling into a region of the first absorbent layer in the planar view. The first absorbent layer includes, in the planar view, an overlapping part that overlaps with a region of the second absorbent layer, and a protruding part that protrudes in the longitudinal direction from the region of the second absorbent layer. The absorbent article is used so that the first absorbent layer is positioned on the side of a user and the second absorbent layer is positioned on the opposite side of the user relative to the first absorbent layer.

With the above-described structure, when the absorbent article is worn, liquid passes through the first absorbent layer and is partly retained by the first absorbent layer. The remaining liquid is absorbed by the second absorbent layer containing the absorbent resin material. The liquid that cannot be absorbed completely by the second absorbent layer can be absorbed by the protruding part of the first absorbent layer protruding from the region of the second absorbent layer in the planar view.

Consequently, leakage of the liquid that cannot be absorbed completely, from the end in the longitudinal direction can be inhibited.

Preferably, the first absorbent layer has a first groove part where the fibrous material is absent, within the region of the first absorbent layer in the planar view. The second absorbent layer has a second groove part where the absorbent resin material is absent, within the region of the second absorbent layer in the planar view.

In this case, by passing through the first groove part, liquid reaches the second absorbent layer faster than when the first groove part is absent. Moreover, when the liquid reaches the second groove part, it is diffused and absorbed into the second absorbent layer faster than when the second groove part is absent. Since the liquid is more easily diffused into the second absorbent layer, the liquid disappears faster from the first absorbent layer, so that irritation to the skin can be more suppressed.

Preferably, the first groove part falls into the region of the second groove part in the planar view.

In this case, since the second groove part of the second absorbent layer is larger than the first groove part of the first absorbent layer, liquid moves from the first absorbent layer to the second absorbent layer in a shorter time than when the second groove part is smaller than the first groove part, so that irritation to the skin can be more suppressed.

Preferably, when the absorbent article 10 is used, part of the protruding part of the first absorbent layer is opposed to an end surface of the second absorbent layer extending in a thickness direction of the second absorbent layer.

In this case, the liquid that cannot be absorbed completely by the second absorbent layer and leaks from the second absorbent layer is immediately absorbed by the protruding part of the first absorbent layer positioned around the second absorbent layer.

Preferably, the first absorbent layer contains the absorbent resin material.

In this case, the amount of liquid that the first absorbent layer absorbs and retains can be increased.

### EFFECTS OF THE INVENTION

The absorbent article of the present invention is capable of inhibiting leakage of the liquid that cannot be absorbed completely, from the end in the longitudinal direction.

### BRIEF DESCRIPTION OF THE DRAWINGS

[Fig. 1] Fig. 1(a) is a schematic cross-sectional view of an absorbent article, Fig. 1(b) is a schematic cross-sectional view taken along the line B-B of Fig. 1(a), and Fig. 1(c) is a schematic cross-sectional view taken along the line C-C of Fig. 1(a) (first embodiment).
[Fig. 2] Fig. 2(a) is a schematic relevant part plan view of the absorbent article, Fig. 2(b) is a schematic side view taken along the line B-B of Fig. 2(a), and Fig. 2(c) is a schematic cross-sectional view taken along the line C-C of Fig. 2(a) (first embodiment).
[Fig. 3] Fig. 3 is a schematic side view of the absorbent article (first embodiment).
[Fig. 4] Fig. 4(a) is a schematic relevant part plan view of an absorbent article, Fig. 4(b) is a schematic side view taken along the line B-B of Fig. 4(a), and Fig. 4(c) is a schematic cross-sectional view taken along the line C-C of Fig. 4(a) (first modification).
[Fig. 5] Fig. 5(a) is a schematic relevant part plan view of an absorbent article, Fig. 5(b) is a schematic side view taken along the line B-B of Fig. 5(a), and Fig. 5(c) is a schematic cross-sectional view taken along the line C-C of Fig. 5(a) (second modification 1).
[Fig. 6] Fig. 6(a) is a schematic relevant part plan view of an absorbent article, Fig. 6(b) is a schematic side view taken along the line B-B of Fig. 6(a), and Fig. 6(c) is a schematic cross-sectional view taken along the line C-C of Fig. 6(a) (second modification 2).
[Fig. 7] Fig. 7(a) is a schematic relevant part plan view of an absorbent article, Fig. 7(b) is a schematic side view taken along the line B-B of Fig. 7(a), and Fig. 7(c) is a schematic cross-sectional view taken along the line C-C of Fig. 7(a) (third modification).
[Fig. 8] Fig. 8(a) is a schematic relevant part plan view of an absorbent article, Fig. 8(b) is a schematic side view taken along the line B-B of Fig. 8(a), and Fig. 8(c) is a schematic cross-sectional view taken along the line C-C of Fig. 8(a) (fourth modification).
[Fig. 9] Fig. 9 is a plan view of the absorbent article (first conventional example).
[Fig. 10] Fig. 10 is a cross-sectional view of the absorbent article (first conventional example).

### MODE FOR CARRYING OUT THE INVENTION

Hereinafter, an embodiment of the present invention will be described with reference to the drawings.

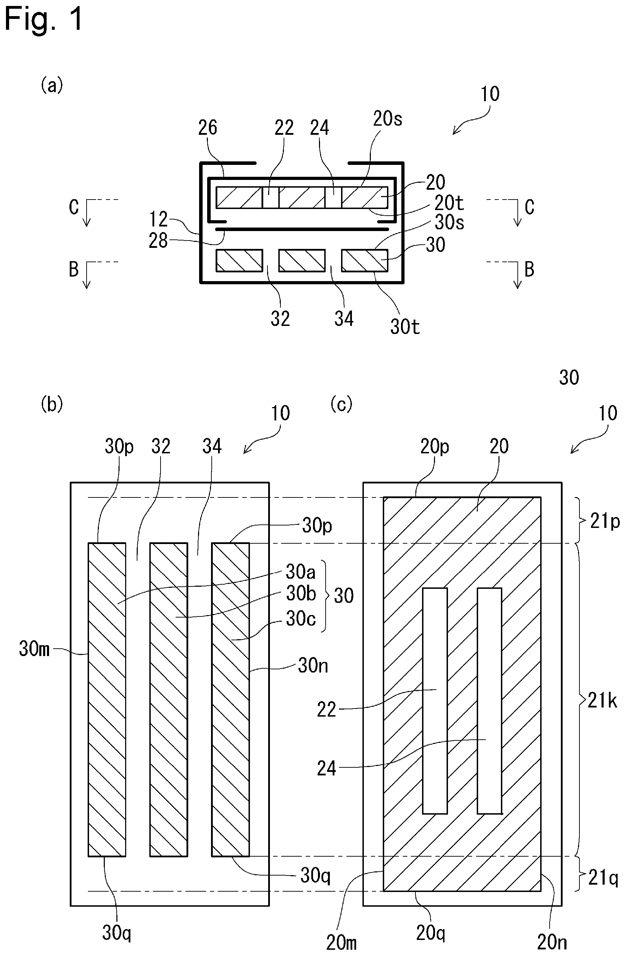

[First embodiment] An absorbent article 10 of a first embodiment will be described with reference to Fig. 1 to Fig. 3.

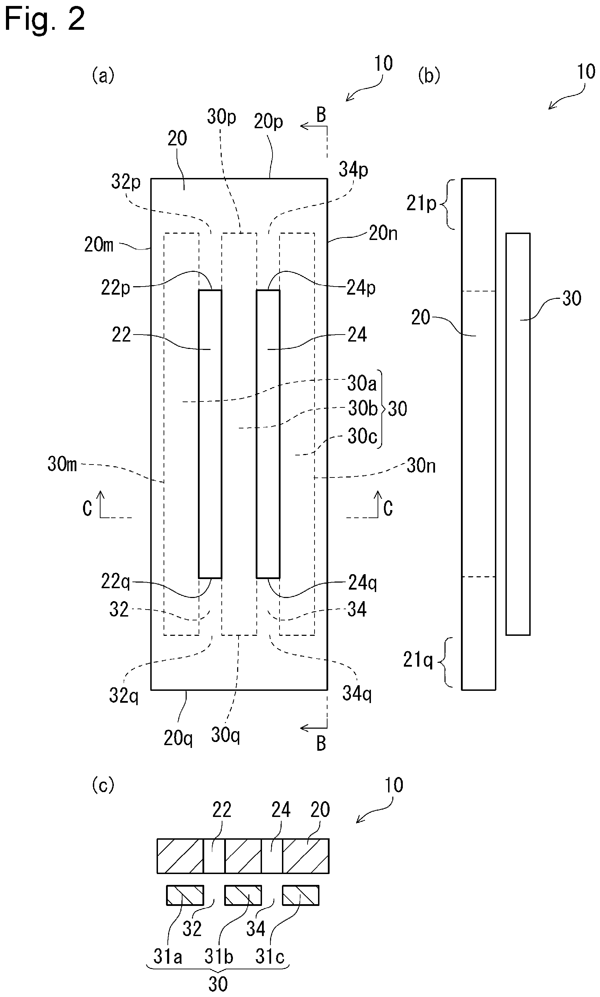

Fig. 1 is a schematic view of the absorbent article 10, and Fig. 2 is a schematic relevant part view of the absorbent article 10. Specifically, Fig. 1(a) is a schematic cross-sectional view of the absorbent article 10, Fig. 1(b) is a schematic cross-sectional view taken along the line B-B of Fig. 1(a), and Fig. 1(c) is a schematic cross-sectional view taken along the line C-C of Fig. 1(a). Fig. 2(a) is a schematic relevant part plan view of the absorbent article 10, Fig. 2(b) is a schematic side view taken along the line B-B of Fig. 2(a), and Fig. 2(c) is a schematic cross-sectional view taken along the line C-C of Fig. 2(a).

As shown in Fig. 1 and Fig. 2, the absorbent article 10 has a first absorbent layer 20 and a second absorbent layer 30 wrapped with a liquid-impermeable exterior sheet 12. The exterior sheet 12 covers the perimeter of the first absorbent layer 20 and covers the entire area of a main surface 30t of the second absorbent layer 30 opposite to the first absorbent layer 20.

The first absorbent layer 20 is formed with a crushed fibrous material, and extends in the longitudinal direction (vertical direction in Figs. 1(b), 1(c), 2(a) and 2(b)). The first absorbent layer 20 is wrapped with, for example, liquid-permeable sheets 26 and 28 (for example, tissue paper). A structure not having the sheet 26 or 28 may be adopted.

The first absorbent layer 20 may contain or not contain an absorbent resin material capable of absorbing and retaining liquid, such as granular or powdery SAP (superabsorbent polymer). In the case where the first absorbent layer 20 contains an absorbent resin material, the amount of liquid that the first absorbent layer 20 absorbs and retains can be increased.

The second absorbent layer 30 is formed with a sheet-like material retaining an absorbent resin material. To form the second absorbent layer 30, for example, a nonwoven fabric sheet is raised to form a bulky structure, an absorbent resin material is sprayed to the bulky structure so that the absorbent resin material is disposed on the surface and inside of the bulky structure, and then, the bulky structure is compressed.

The first absorbent layer 20 and the second absorbent layer 30 are disposed so as to overlap with each other in the planar view.

Specifically, as shown in Figs. 1(b), 1(c) and 2(a), the second absorbent layer 30 falls into the region of the first absorbent layer 20 in the planar view. That is, the second absorbent layer 30 is disposed so as to fall into a region surrounded by end edges 20p and 20q and side edges 20m and 20n of the first absorbent layer 20 in the planar view.

The first absorbent layer 20 includes, in the planar view, an overlapping part 21k that overlaps with the region of the second absorbent layer 30, and protruding parts 21p and 21q that protrude in the longitudinal direction from the region of the second absorbent layer 30. That is, in the planar view, the end edges 20p and 30p on one sides of the first absorbent layer 20 and the second absorbent layer 30 in the longitudinal direction are away from each other, and the end edges 20q and 30q on the other sides of the first absorbent layer 20 and the second absorbent layer 30 in the longitudinal direction are away from each other.

Further, in the planar view, the side edges 20m and 30m on one sides of the first absorbent layer 20 and the second absorbent layer 30 in the width direction are away from each other, and the side edges 20n and 30n on the other sides in the width direction are away from each other. In the planar view, the side edges 20m and 30m on one sides in the width direction may overlap with each other, and the side edges 20n and 30n on the other sides in the width direction may overlap with each other.

The first absorbent layer 20 has, in the planar view, first groove parts 22 and 24 where no fibrous material is present, within the region of the first absorbent layer 20. While a case is shown where the first groove parts 22 and 24 pass through the opposing main surfaces 20s and 20t of the first absorbent layer 20, they may be bottomed grooves. The first groove parts 22 and 24 extend parallel to each other in the longitudinal direction of the first absorbent layer 20, and in the planar view, the entire perimeters of the first groove parts 22 and 24 are surrounded by the first absorbent layer 20.

In the second absorbent layer 30, a plurality of element parts 30a to 30c which are formed with a sheet-like material retaining an absorbent resin material and extend in the longitudinal direction are arranged with spacing between them in the width direction, and second groove parts 32 and 34 are formed between the adjoining element parts 30a and 30b, and 30b and 30c. That is, the second absorbent layer 30 has, in the planar view, the second groove parts 32 and 34 where no absorbent resin material is present, within the region of the second absorbent layer 30. The second groove parts 32 and 34 extend in the longitudinal direction, and both ends 32p and 32q, and 34p and 34q of the second groove parts 32 and 34 in the longitudinal direction are aligned with the end edges 30p and 30q of the second absorbent layer 30.

The second absorbent layer 30 may be integrally formed, for example, by arranging the element parts 30a to 30c with a spacing between them in the width direction on a non-illustrated base sheet and bonding them.

The first groove parts 22 and 24 and the second groove parts 32 and 34 overlap with each other in the planar view, and the first groove parts 22 and 24 fall into the regions of the second groove parts 32 and 34. Specifically, in the planar view, the second groove parts 32 and 34 include intermediate parts overlapping with the first groove parts 22 and 24 and extension parts extending from both sides of the intermediate parts. One ends 22p and 24p of the first groove parts 22 and 24 in the longitudinal direction and one ends 32p and 34p of the second groove parts 32 and 34 in the longitudinal direction are away from each other, and the other ends 22q and 24q of the first groove parts 22 and 24 in the longitudinal direction and the other ends 32q and 34q of the second groove parts 32 and 34 in the longitudinal direction are away from each other.

In the planar view, the first groove parts 22 and 24 and the second groove parts 32 and 34 overlap with each other in the width direction, and the side edges thereof overlap with each other.

The absorbent article 10 may be used for wearable articles such as paper diapers, underpants-type diapers and sanitary napkins. The absorbent article 10 is used so that the first absorbent layer 20 is positioned on the user's side and the second absorbent layer 30 is positioned on the opposite side of the user relative to the first absorbent layer 20. When the wearable article is worn, the absorbent article 10 has its one end in the longitudinal direction disposed on the user' lower abdomen and has its other end in the longitudinal direction disposed on the side of the user's buttocks.

Liquid moves from the overlapping part 21k in an intermediate position of the first absorbent layer 20 of the absorbent article 10 in the longitudinal direction to the second absorbent layer 30. At this time, part of the liquid is retained by the first absorbent layer 20, and the remaining liquid passes through the first absorbent layer 20 to be absorbed by the second absorbent layer 30.

The liquid that cannot be absorbed completely and leaks from the second absorbent layer 30 can be absorbed by the protruding parts 21p and 21q of the first absorbent layer 20 protruding from the second absorbent layer 30 in the longitudinal direction in the planar view. This enables the absorbent article 10 to inhibit leakage of the liquid that cannot be absorbed completely, from the ends in the longitudinal direction.

The displacement of the first absorbent layer 20 when liquid is absorbed can be made smaller than that of the second absorbent layer 30. For this reason, when the first absorbent layer 20 is positioned on the user's side, compared with when the second absorbent layer 30 is positioned on the user's side, irritation caused to the user when liquid is absorbed can be made small to mitigate user discomfort.

By passing through the first groove parts 22 and 24, liquid reaches the second absorbent layer 30 faster than when the first groove parts 22 and 24 are absent. Moreover, liquid is diffused from the second groove parts 32 and 34 and absorbed into the second absorbent layer 30 faster than when the second groove parts are absent, and flows out from the one ends 32p and 34p and/or the other ends 32q and 34q of the second groove parts 32 and 34 to be absorbed by the protruding parts 21p and/or 21q of the first absorbent layer 20 protruding from the second absorbent layer 30 in the longitudinal direction in the planar view. Because this makes the liquid disappear faster from the first absorbent layer 20 on the user's skin surface side, irritation to the skin can be more suppressed.

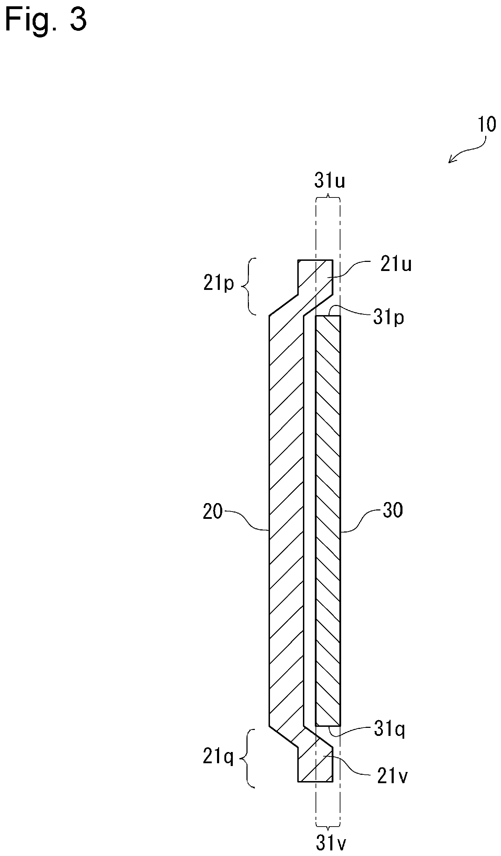

Fig. 3 is a schematic side view of the absorbent article 10 when the absorbent article 10 is used. When the user wears the absorbent article 10, the first absorbent layer 20 is pressed against the non-skin surface side from the skin surface side of the user. At this time, parts 21u and 21v of the protruding parts 21p and 21q of the first absorbent layer 20 protruding from the second absorbent layer 30 in the longitudinal direction in the planar view are opposed to end surfaces 31p and 31q of the second absorbent layer 30 extending in the thickness direction of the second absorbent layer 30. That is, the parts 21u and 21v of the protruding parts 21p and 21q of the first absorbent layer 20 are positioned within opposing regions 31u and 31v opposed to the end surfaces 31p and 31q of the second absorbent layer 30. In this case, the liquid that cannot be absorbed completely by the second absorbent layer 30 and leaks from the second absorbent layer 30 is immediately absorbed by the protruding parts 21p and 21q of the first absorbent layer 20 positioned around the second absorbent layer 30.

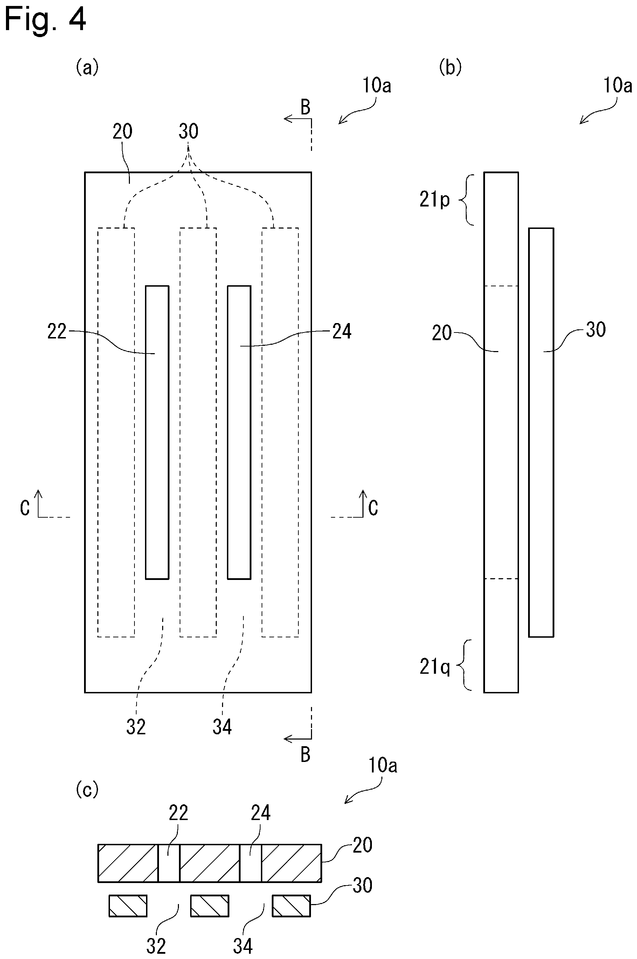

Next, various modifications of the first embodiment will be described with reference to Fig. 4 through Fig. 8 which are schematic relevant part views similar to Fig. 2. In the following, component parts similar to those of the first embodiment will be denoted by the same reference numerals and differences from the first embodiment will be mainly described.

[First modification] While in the first embodiment, the boundary lines of the regions of the first groove parts 22 and 24 and the boundary lines of the regions of the second groove parts 32 and 34 are arranged so as to overlap with each other in the planar view, like an absorbent article 10a of a first modification shown in Fig. 4, the boundary lines of the regions of the first groove parts 22 and 24 and the boundary lines of the regions of the second groove parts 32 and 34 may be arranged so as not to overlap at all. That is, as shown in Fig. 4(a), the first groove parts 22 and 24 may be arranged, in the planar view, so as to fall into the regions of the second groove parts 32 and 34 with a spacing from the regions of the second groove parts 32 and 34 over the entire perimeter.

With the absorbent article 10a of the first modification, since the first groove parts 22 and 24 fall into the regions of the second groove parts 32 and 34 in the planar view, as in the first embodiment, liquid disappears faster from the first absorbent layer on the user's skin surface side, so that irritation to the skin can be more suppressed.

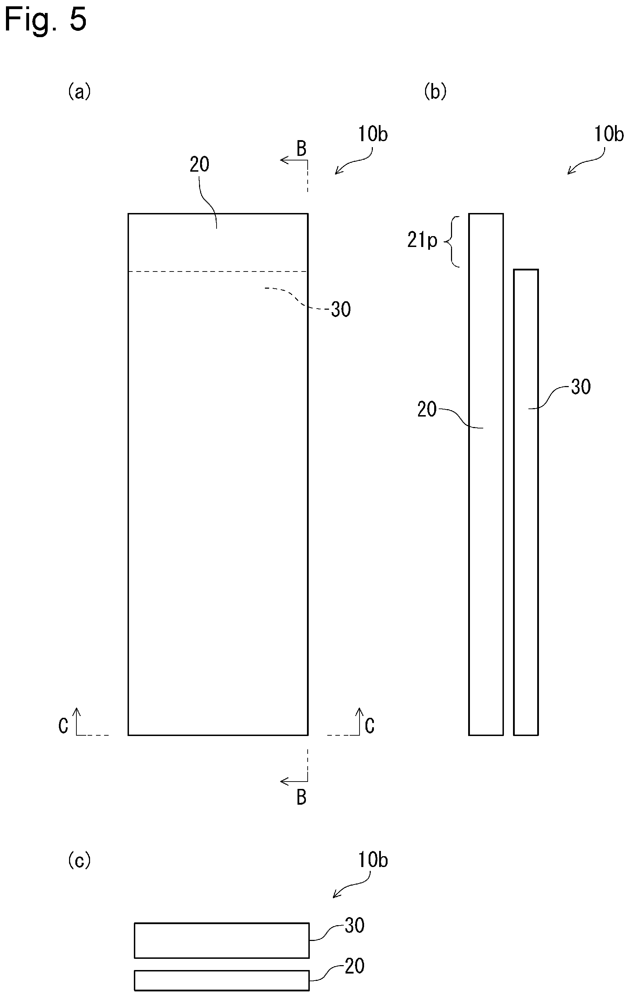

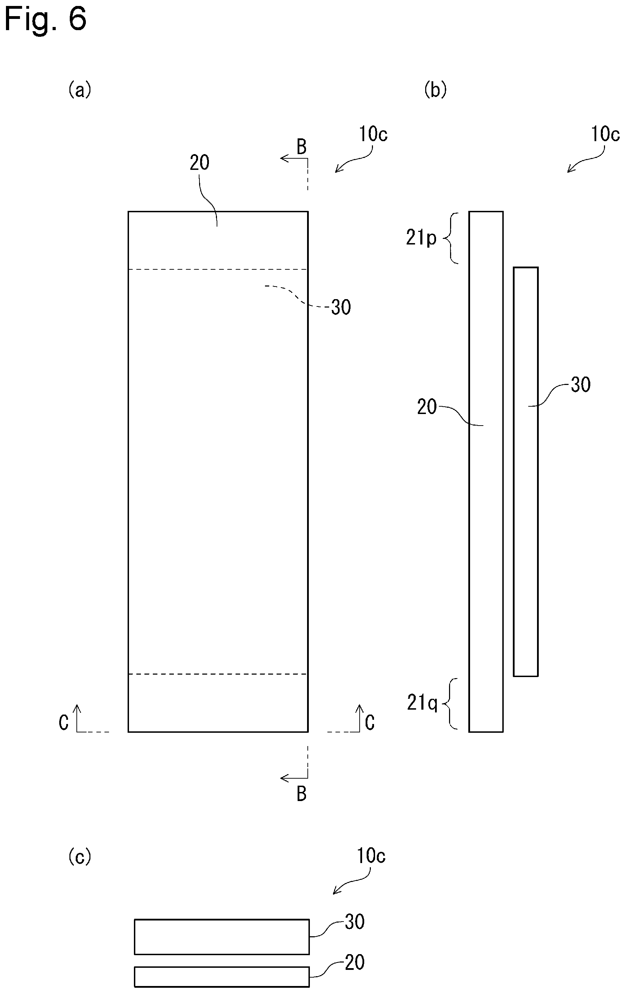

[Second modification] While in the first embodiment, the first absorbent layer 20 has the first groove parts 22 and 24 and the second absorbent layer 30 has the second groove parts 32 and 34, like absorbent articles 10b and 10c of a second modification shown in Fig. 5 and Fig. 6, the first groove parts 22 and 24 and the second groove parts 32 and 34 may be absent.

The first absorbent layer 20 of the absorbent article 10b shown in Fig. 5 includes the protruding part 21p protruding from the region of the second absorbent layer 30 toward one side in the longitudinal direction in the planar view, and does not protrude from the region of the second absorbent layer 30 toward the other side in the longitudinal direction.

The first absorbent layer 20 of the absorbent article 10c shown in Fig. 6 includes the protruding part 21p protruding from the region of the second absorbent layer 30 toward one side in the longitudinal direction and the protruding part 21q protruding toward the other side in the longitudinal direction in the planar view.

With the absorbent articles 10b and 10c, since liquid that cannot be absorbed completely by the second absorbent layer 30 and leaks from the second absorbent layer 30 is absorbed by the protruding parts 21p and/or 21q of the first absorbent layer 20, leakage of the liquid that cannot be absorbed completely, from the ends in the longitudinal direction can be inhibited.

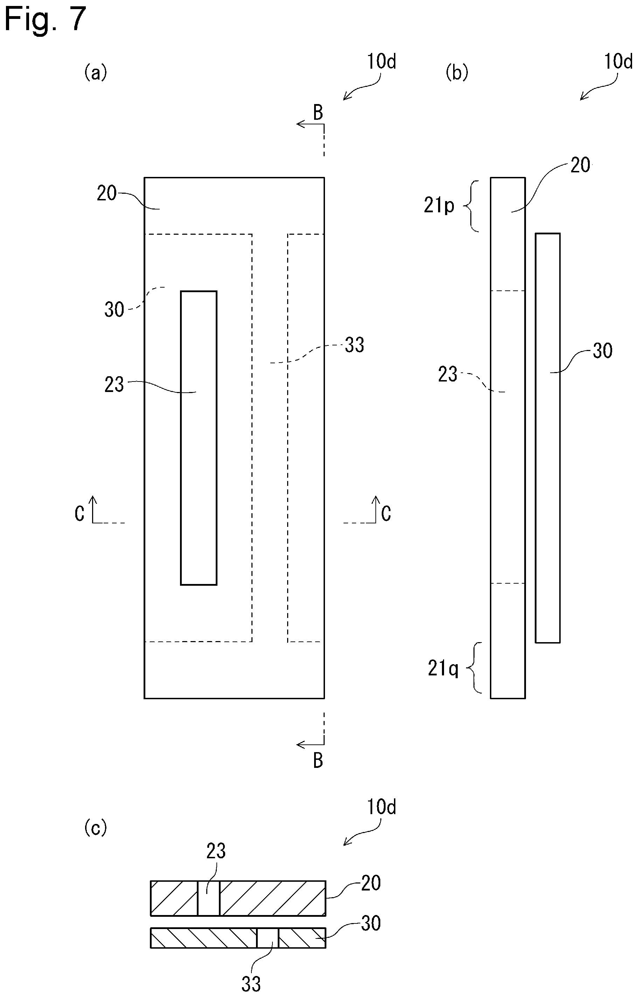

[Third modification] Like an absorbent article 10d of a third modification shown in Fig. 7, a first groove part 23 of the first absorbent layer 20 may be disposed outside the region of a second groove part 33 of the second absorbent layer 30.

In the absorbent article 10d, in the planar view, the second absorbent layer 30 is disposed within the region of the first groove part 23. Since liquid is absorbed in a shorter time and disappears faster from the first absorbent layer 20 on the user's skin surface side than when the first absorbent layer 20 does not have the first groove part 23, irritation to the skin can be more suppressed. Since liquid that cannot be absorbed completely by the second absorbent layer 30 can be absorbed by the protruding parts 21p and 21q of the first absorbent layer 20 protruding from the region of the second absorbent layer 30 in the planar view, leakage of the liquid that cannot be absorbed completely, from the ends in the longitudinal direction can be inhibited.

More than one first groove part 23 and/or second groove part 33 may be provided.

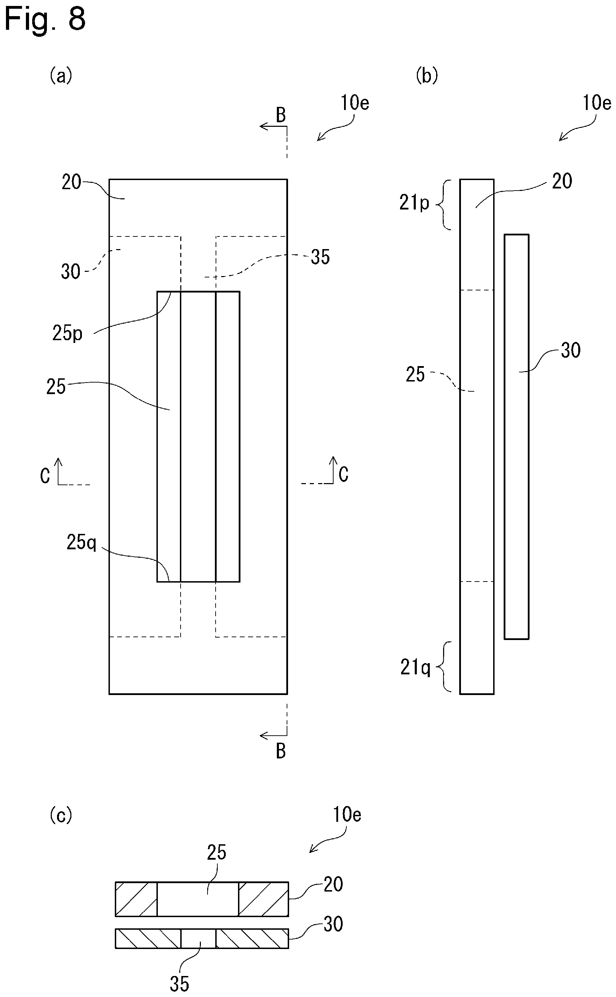

[Fourth modification] Like an absorbent article 10e of a fourth modification shown in Fig. 8, a first groove part 25 may be disposed so as to protrude outward in the width direction of the region of a second groove part 35 in the planar view.

In the absorbent article 10e, the second absorbent layer 30 and the second groove part 35 are disposed within the region of the first groove part 25 in the planar view. Since liquid is absorbed in a shorter time and disappears faster from the first absorbent layer 20 on the user's skin surface side than when the first absorbent layer 20 does not have the first groove part 25, irritation to the skin can be more suppressed. Since liquid that cannot be absorbed completely by the second absorbent layer 30 can be absorbed by the protruding parts 21p and 21q of the first absorbent layer 20 protruding from the region of the second absorbent layer 30 in the planar view, leakage of the liquid that cannot be absorbed completely, from the ends in the longitudinal direction can be inhibited.

More than one first groove part 25 and/or second groove part 35 may be provided.

[Summary] As described above, the absorbent articles 10, 10a to 10e are capable of inhibiting leakage of liquid that cannot be absorbed completely, from the ends in the longitudinal direction.

The present invention is not limited to the above-described embodiment but may be carried out with various modifications being added.

For example, in the planar view, the shapes of the absorbent article, the first absorbent layer, the second absorbent layer, the first groove part and the second groove part are not limited to the illustrated rectangular shapes but may be any appropriate shapes.

Moreover, the structures disclosed in the first embodiment and the first to fourth modifications may be appropriately selected and combined to form an absorbent article. For example, a structure may be adopted in which, of a plurality of first groove parts, some are disposed within the region of the second groove part and the others are disposed outside the second groove part.

### DESCRIPTION OF REFERENCE NUMERALS

10, 10a to 10e Absorbent article
20 First absorbent layer
21k Overlapping part
21p, 21q Protruding part
22, 23, 24, 25 First groove part
30 Second absorbent layer
31p, 31q End surface
32, 33, 34, 35 Second groove part

## Claims

1. An absorbent article comprising:
a first absorbent layer formed with a crushed fibrous material and extending in a longitudinal direction; and
a second absorbent layer formed with a sheet-like material retaining an absorbent resin material and falling into a region of the first absorbent layer in the planar view,
wherein the first absorbent layer includes, in the planar view, an overlapping part that overlaps with a region of the second absorbent layer, and a protruding part that protrudes in the longitudinal direction from the region of the second absorbent layer, and
the absorbent article is used so that the first absorbent layer is positioned on the side of a user and the second absorbent layer is positioned on the opposite side of the user relative to the first absorbent layer.

2. The absorbent article according to claim 1, wherein the first absorbent layer has a first groove part where the fibrous material is absent, within the region of the first absorbent layer in the planar view, and
the second absorbent layer has a second groove part where the absorbent resin material is absent, within the region of the second absorbent layer in the planar view.

3. The absorbent article according to claim 2, wherein the first groove part falls into the region of the second groove part in the planar view.

4. The absorbent article according to claims 1 to 3, wherein when the absorbent article 10 is used, part of the protruding part of the first absorbent layer is opposed to an end surface of the second absorbent layer extending in a thickness direction of the second absorbent layer.

5. The absorbent article according to any one of claims 1 to 4, wherein the first absorbent layer contains the absorbent resin material.
